# EUROPEAN PATENT SPECIFICATION

(11) **EP 3 903 894 B1**
(45) Date of publication and mention of the grant of the patent: **10.09.2025**
(21) Application number: 19903142.8
(22) Date of filing: 27.12.2019
(51) Int. Cl.: A63B 69/00, G01K 1/02, G01K 7/00, A61F 7/00, A61B 5/01, A61B 5/00, A61N 2/00, G01K 13/20

(54) **MOTION ASSISTANCE PROGRAM, MOTION ASSISTANCE SYSTEM, AND MOTION ASSISTANCE SYSTEM CONTROL METHOD**
PROGRAMM ZUR BEWEGUNGSUNTERSTÜTZUNG, SYSTEM ZUR BEWEGUNGSUNTERSTÜTZUNG UND VERFAHREN ZUR BEWEGUNGSUNTERSTÜTZUNG
PROGRAMME D'AIDE AU MOUVEMENT, SYSTÈME D'AIDE AU MOUVEMENT ET PROCÉDÉ DE COMMANDE DE SYSTÈME D'AIDE AU MOUVEMENT

(30) Priority: 27.12.2018 JP 2018246252
(43) Date of publication of application: 03.11.2021
(73) Proprietor: Panasonic Intellectual Property Management Co., Ltd., Osaka-shi, Osaka 540-6207 (JP)
(72) Inventor: YABUUCHI, Rise, Osaka-shi, Osaka 540-6207 (JP); INUI, Keita, Osaka-shi, Osaka 540-6207 (JP); SHINOMIYA, Yoichi, Osaka-shi, Osaka 540-6207 (JP)
(74) Representative: Grünecker Patent- und Rechtsanwälte PartG mbB
(86) International application number: PCT/JP2019/051508
(87) International publication number: WO 2020/138453

(56) References cited:
- JP-A- 2000 245 863
- JP-A- 2008 246 196
- JP-A- 2010 516 407
- JP-A- 2018 068 478
- US-A1- 2007 060 987
- US-A1- 2015 297 909
- US-A1- 2017 239 080
- US-A1- 2018 125 703
- MORRIS CASSANDRA ET AL: "Static magnetic fields alter arteriolar tone in vivo", BIOELECTROMAGNETICS, vol. 26, no. 1, 1 January 2004 (2004-01-01), US, pages 1 - 9, XP055873653, ISSN: 0197-8462, DOI: 10.1002/bem.20047
- TOKIZAWA, KEN: "Trends and countermeasures of heatstroke in workplace", REITO - REFRIGERATION, vol. 93, no. 1093, 15 November 2018 (2018-11-15), pages 10 (732) - 14 (736), XP009528685, ISSN: 0034-3714
- GRAHN A. DENNIS: "WORK VOLUME AND STRENGTH TRAINING RESPONSES TO RESISTIVE EXERCISE IMPROVE WITH PERIODIC HEAT EXTRACTION FROM THE PALM", JOURNAL OF STRENGTH AND CONDITIONING RESEARCH, vol. 26, no. 9, September 2012 (2012-09-01), pages 2558 - 2569, XP055722879, DOI: 10.1519/JSC.0b013e31823f8c1a

## Description

The present invention relates to an exercise assistance program, an exercise assistance system, and an exercise assistance system control method.

A known exercise assistance system distinguishes different types of walking actions. A conventional exercise assistance system assists, for example, exercising of the legs. Patent document 1 discloses one example of a conventional exercise assistance system.

Patent Document 1: Japanese Laid-Open Patent Publication No. 2015-136582

US 2007/060987 A1) relates to a system for transferring heat to/from a core portion of the human body. A core temperature of the user is measured. Based on the measured temperature, the core temperature of the body is lowered to below a threshold by cooling the hands and feet of the user.

US 2017/0239080 A1 relates to a system for cooling or warming a mammal, wherein the blood flow through a certain portion of the body is increased. This may be performed by applying a certain level of under-pressure or by positioning magnets on or near the desired body area. Problems that the Invention is to Solve

It is understood that information related to deep body temperature can be used to manage exercising performance. Deep body temperature cannot be measured with the conventional exercise assistance system.

It is an objective of the present invention to provide an exercise assistance program, an exercise assistance system, and an exercise assistance system control method that allow for the measurement of deep body temperature.

The invention is defined by the independent claims. The dependent claims describe advantageous embodiments.

One aspect of an exercise assistance program in accordance with the present invention has a controller execute a first step of reading first state information related to a deep body temperature detected by a detector, a second step of calculating the deep body temperature from the first state information, and a third step of outputting second state information related to the calculated deep body temperature.

One aspect of an exercise assistance system in accordance with the present invention includes an attaching portion attached to an auricle, a detector arranged on the attaching portion, a transmitter that transmits the first state information detected by the detector, and the exercise assistance program.

### Effects of the Invention

The exercise assistance program, exercise assistance system, and exercise assistance system control method according to the present invention allow for the measurement of deep body temperature.

### BRIEF DESCRIPTION OF THE DRAWINGS

Fig. 1 is a perspective view showing one usage example of an exercise assistance system in accordance with one embodiment.
Fig. 2 is a perspective view showing a deep body thermometer illustrated in Fig. 1.
Fig. 3 is a perspective view showing a temperature adjustor illustrated in Fig. 1.
Fig. 4 is front view showing the palm of a mitten illustrated in Fig. 3.
Fig. 5 is front view showing the sole of a boot illustrated in Fig. 3.
Fig. 6 is a block diagram of the exercise assistance system illustrated in Fig. 1.
Fig. 7 is a graph showing one example of how the deep body temperature changes during training.
Fig. 8 is a graph showing one example of how the deep body temperature changes when using the temperature adjustor.
Fig. 9 is a flowchart showing one example of a first control executed with an exercise assistance program.
Fig. 10 is a flowchart showing one example of a second control executed with the exercise assistance program.
Fig. 11 is a flowchart showing one example of how to use the exercise assistance system.

### MODES FOR CARRYING OUT THE INVENTION

Examples of aspects of an exercise assistance program, an exercise assistance system, and an exercise assistance system control method
A first aspect of an exercise assistance system in accordance with the present invention includes a deep body thermometer attached to a user and including a temperature sensor, temperature adjustment equipment attached to at least one of a hand and a foot of the user, and a controller configured to communicate with the deep body thermometer and the temperature adjustment equipment. The deep body thermometer is configured to detect information related to deep body temperature of the user with the temperature sensor. The controller is configured to execute a process for determining whether the deep body temperature of the user is less than or equal to a predetermined temperature based on the detected information related to deep body temperature. When the controller determines that the deep body temperature is not less than or equal to the predetermined temperature, the controller is configured to execute a process for outputting control information to the temperature adjustment equipment indicating a magnetic stimulating pattern that dilates blood vessels in a palm or foot bottom of the user.

In a first aspect of a method for controlling an exercise assistance system in accordance with the present invention, the exercise assistance system includes a deep body thermometer, which is attached to a user and includes a temperature sensor, and temperature adjustment equipment, which is attached to at least one of a hand and a foot of the user. The method includes detecting information related to deep body temperature of the user with the temperature sensor; determining whether the deep body temperature of the user is less than or equal to a predetermined temperature based on the detected information related to deep body temperature; and when determined that the deep body temperature is not less than or equal to the predetermined temperature, outputting control information indicating a magnetic stimulating pattern that dilates blood vessels in a palm or foot bottom of the user.

In a first aspect of an exercise assistance program in accordance with the present invention, the exercise assistance program has a controller of a terminal device execute a process for detecting information related to deep body temperature of a user with a temperature sensor arranged in a deep body thermometer attached to the user; a process for determining whether the deep body temperature of the user is less than or equal to a predetermined temperature based on the detected information related to deep body temperature; and when determined that the deep body temperature is not less than or equal to the predetermined temperature, a process for outputting control information to temperature adjustment equipment attached to at least one of a hand and a foot of the user. The control information indicates a magnetic stimulating pattern that dilates blood vessels in a palm or foot bottom of the user

In a second aspect of method for controlling an exercise assistance system in accordance with the present invention, the method includes continuously obtaining, via a network, first information indicating deep body temperature of a user that is detected with a deep body thermometer attached to the user; receiving second information related to time for lowering the deep body temperature of the user to a predetermined temperature or less; and determining control information for temperature adjustment equipment to lower the deep body temperature of the user to the predetermined temperature or less within the time based on the first information and the second information. The temperature adjustment equipment is configured to be attached to at least one of a hand and a foot of the user to dilate blood vessels in a palm or foot bottom of the user. The method further includes outputting the determined control information to the temperature adjustment equipment via the network.

In a second aspect of an exercise assistance program in accordance with the present invention, the exercise assistance program has a controller of a terminal device execute a process for continuously obtaining, via a network, first information indicating deep body temperature of a user that is detected with a deep body thermometer attached to the user; a process for receiving second information related to time for lowering the deep body temperature of the user to a predetermined temperature or less; a process for determining control information for temperature adjustment equipment to lower the deep body temperature of the user to the predetermined temperature or less within the time based on the first information and the second information, wherein the temperature adjustment equipment is configured to be attached to at least one of a hand and a foot of the user to dilate blood vessels in a palm or foot bottom of the user; and a process for outputting the determined control information to the temperature adjustment equipment via the network.

In one example of the second aspect of the method for controlling an exercise assistance system, the temperature adjustment equipment includes at least one of a cooling device, which includes a cooling medium for cooling one of a hand and a foot of the user, and a magnetic device, which applies magnetic stimuli to the user to dilate blood vessels of the user. The control information includes at least one of a temperature of the cooling medium in the cooling device, a magnetic stimulating pattern applied to the user by the magnetic device, and a driving time of the temperature adjustment equipment.

In a third aspect of an exercise assistance program in accordance with the present invention, the exercise assistance program has a controller of a terminal device execute a process for detecting information related to deep body temperature of a user with a temperature sensor arranged in a deep body thermometer attached to the user; a process for determining whether the deep body temperature of the user, which is calculated from the detected information related to deep body temperature, is less than or equal to a predetermined temperature; and a process for performing, when determined that the deep body temperature is not less than or equal to the predetermined temperature, (i) applying magnetic stimuli to at least one of a palm and foot bottom of the user, (ii) cooling at least one of the palm and the foot bottom of the user, or performing both of (i) and (ii).

A further aspect of an exercise assistance program in accordance with the present invention has a controller execute a first step of reading first state information related to a deep body temperature detected by a detector, a second step of calculating the deep body temperature from the first state information, and a third step of outputting second state information related to the calculated deep body temperature.

The exercise assistance program allows the deep body temperature to be measured.

One example of the exercise assistance program further has the controller execute a fourth step of calculating first instruction information from the second state information to control a temperature adjustor that adjusts a body temperature. The third step further outputs the first instruction information.

The exercise assistance program allows the body temperature to be adjusted in accordance with the calculated deep body temperature.

One example of the exercise assistance program calculates, in the fourth step, the first instruction information to operate the temperature adjustor so that the deep body temperature will be included in the predetermined temperature range.

The exercising performance is correlated to the deep body temperature. When the deep body temperature is included in the predetermined temperature range, the exercising performance will be improved. The exercise assistance program improves the exercising performance.

In one example of the exercise assistance program, the first instruction information is calculated to operate the temperature adjustor so that the deep body temperature will be included in the predetermined temperature range within a predetermined period in the fourth step.

The exercise assistance program allows the deep body temperature to be included in the predetermined temperature range within a limited length of time.

One example of the exercise assistance program further has the controller execute a fifth step of calculating second instruction information from the second state information to control a notification unit that issues a notification of information related to a body temperature. The fifth step further outputs the second instruction information.

The exercise assistance program presents the person who is exercising and his or her trainer or the like with information related to the calculated deep body temperature.

In one example of the exercise assistance program, a predicted deep body temperature is calculated from the first state information in the second step.

The exercise assistance program presents the person who is exercising and his or her trainer or the like with information related to the predicted deep body temperature.

In one example of the exercise assistance program, an exercise-related performance is calculated from the predicted deep body temperature in the second step.

The exercise assistance program presents the person who is exercising and his or her trainer or the like with exercise-related information that is based on the deep body temperature.

One aspect of an exercise assistance system in accordance with the present invention includes an attaching portion attached to an auricle, a detector arranged on the attaching portion, a transmitter that transmits the first state information detected by the detector, and the exercise assistance program.

The exercise assistance system allows the deep body temperature to be measured.

One example of the exercise assistance system further includes a receiver, which receives information transmitted from a controller that executes the exercise assistance program, and a temperature adjustor, which adjusts body temperature in accordance with the received information.

The exercise assistance system allows the body temperature to be adjusted in accordance with the calculated deep body temperature.

### Embodiment

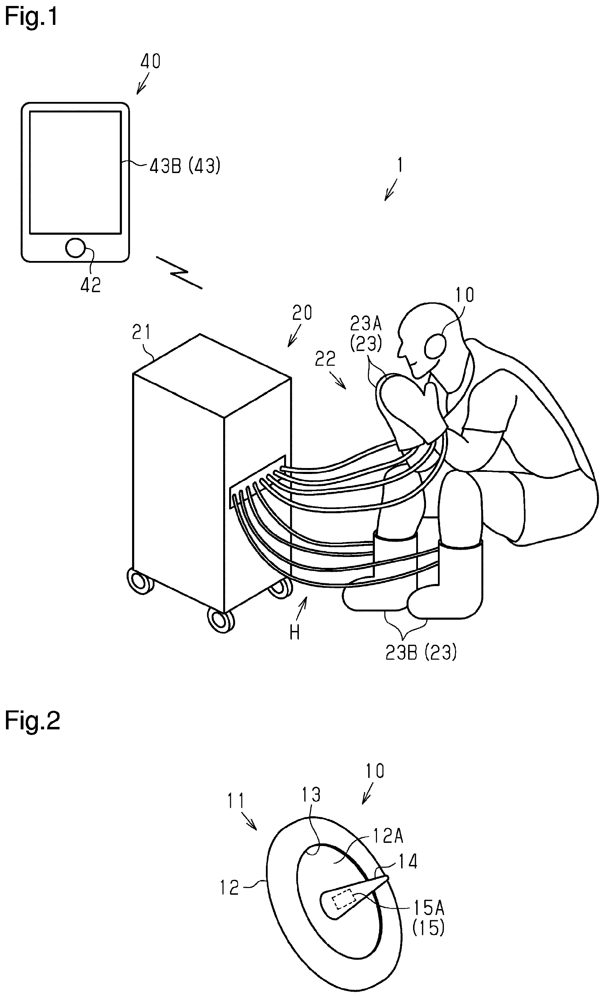

With reference to Fig. 1, the configuration of an exercise assistance system 1 will now be described.

The exercise assistance system 1 is used, for example, during training or in a training interval by a person who is exercising. Training includes exercises related to normal daily activities, sports, and the like. The main elements of the exercise assistance system 1 include a deep body thermometer 10, a temperature adjustor 20, a terminal device 40, and an exercise assistance program.

The deep body thermometer 10 is configured to measure deep body temperature T. The deep body temperature T is the temperature inside the body. The inside of the body includes, for example, the brain, the internal organs, and the like. The deep body thermometer 10 is configured to be electrically connectable to the terminal device 40 through wired communication or wireless communication. In one example, the deep body thermometer 10 is configured to be connectable to the terminal device 40 through wireless communication.

The temperature adjustor 20 is configured to adjust the temperature of the body. The temperature of the body includes, for example, body surface temperature. A change in the body temperature affects the deep body temperature T. Thus, adjustment of the body temperature allows for adjustment of the deep body temperature T. The temperature adjustor 20 is configured to be electrically connectable to the terminal device 40 through wired or wireless communication. In one example, the temperature adjustor 20 is configured to be connectable to the terminal device 40 through wireless communication.

The terminal device 40 is separate from the deep body thermometer 10 and the temperature adjustor 20. The terminal device 40 includes at least one of a smart device and a personal computer. A smart device includes at least one of a smartphone, a table computer, and a wearable device such as a smart watch. In the example illustrated in Fig. 1, the terminal device 40 includes a tablet computer. The terminal device 40 is paired with the deep body thermometer 10 and the temperature adjustor 20 for connection with the deep body thermometer 10 and the temperature adjustor 20 through wireless communication. The deep body thermometer 10, the temperature adjustor 20, and the terminal device 40 each include communication elements required for pairing. The terminal device 40 can be attached to the temperature adjustor 20.

With reference to Fig. 2, the structure of the deep body thermometer 10 will now be described.

The deep body thermometer 10 includes an attaching portion 11 attached to an auricle. The main elements of the attaching portion 11 include a main body 12, a hanging portion 13, and an insertion portion 14. The main body 12 accommodates various types of electrical elements of the deep body thermometer 10. The hanging portion 13 is configured to be hung on the auricle. The hanging portion 13 is arranged on an inner surface 12A of the main body 12. The insertion portion 14 is configured to be insertable into the earhole. The insertion portion 14 has, for example, a tapered shape. The insertion portion 14 is arranged on the inner surface 12A of the main body 12. In one example, the insertion portion 14 is inserted into the earhole, and the hanging portion 13 is hung on the auricle to attach the attaching portion 11 to the auricle. The deep body temperature T is measured in a state in which the attaching portion 11 is attached to the auricle.

The deep body thermometer 10 further includes a detector 15 that detects the deep body temperature T. The detector 15 is arranged in the attaching portion 11. In one example, the detector 15 is arranged in the insertion portion 14. The detector 15 includes a temperature sensor 15A. The temperature sensor 15A includes at least one of a temperature detection element that detects the ambient temperature and an infrared detection element that detects infrared emitted from the eardrum or its periphery. One example of a temperature detection device is a thermistor. One example of an infrared detection element is a thermopile. In one example, the deep body temperature T is measured based on the detection result of the thermistor and the detection result of the thermopile.

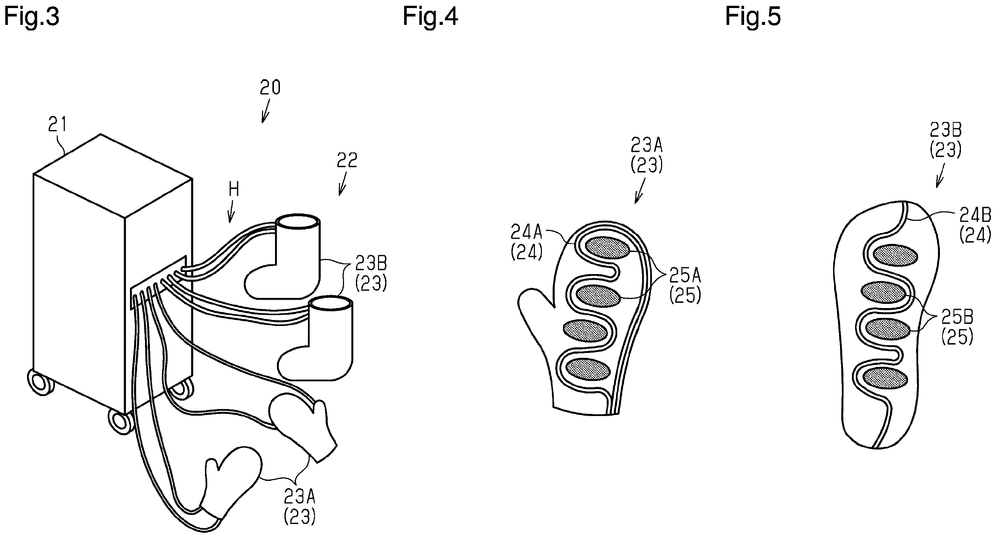

With reference to Fig. 3, the configuration of the temperature adjustor 20 will now be described.

The main elements of the temperature adjustor 20 include a main body 21 and adjustment equipment 22. The main body 21 accommodates various types of electrical elements of the temperature adjustor 20. The main body 21 is configured to be movable on, for example, a plane surface. One example of the plane surface is ground surface. The adjustment equipment 22 is configured to be suited for adjusting the body temperature. The adjustment equipment 22 includes one or more attaching members 23. Preferably, the subject to which the attaching members 23 are attached include the periphery of the body. In the example illustrated in Fig. 3, the attaching members 23 include a pair of mittens 23A and a pair of boots 23B. The adjustment equipment 22 further includes a cooling device 24 and a magnetic device 25 (refer to Fig. 4). The cooling device 24 is configured to supply, for example, a cooling medium that cools the body. Hoses, which supply the cooling medium to the cooling device 24, connect the main body 21 and the attaching members 23. A tank (not shown) storing the cooling medium is incorporated in, for example, the main body 21. The magnetic device 25 is configured to apply magnetic stimuli that dilates blood vessels.

As shown in Fig. 4, the cooling device 24 and the magnetic device 25 are arranged on the mittens 23A. The cooling device 24 includes a passage 24A through which the cooling medium flows. In one example, the passage 24A of the cooling device 24 is arranged on the mittens 23A to supply the cooling medium to the palms. The magnetic device 25 includes stimulating portions 25A that apply magnetic stimuli. In one example, the stimulating portions 25A of the magnetic device 25 are arranged on the mittens 23A to apply magnetic stimuli to the palms. In the example shown in Fig. 4, the passage 24A of the cooling device 24 meanders between the stimulating portions 25A of the magnetic device 25. In a state in which the mittens 23A are attached to the hands, activation of the cooling device 24 and the magnetic device 25 lowers the temperature of the hands. This, in turn, lowers the deep body temperature T.

As shown in Fig. 5, the cooling device 24 and the magnetic device 25 are arranged on the boots 23B. The cooling device 24 includes a passage 24B through which the cooling medium flows. In one example, the passage 24B of the cooling device 24 is arranged on the soles of the boots 23B to supply the cooling medium to the bottom of the feet. The magnetic device 25 includes one or more stimulating portions 25B to apply magnetic stimuli. In one example, the stimulating portions 25B of the magnetic device 25 are arranged on the soles of the boots 23B. In the example shown in Fig. 5, the passage 24B of the cooling device 24 meanders between the stimulating portions 25B of the magnetic device 25. In a state in which the boots 23B are attached to the feet, activation of the cooling device 24 and the magnetic device 25 lowers the temperature of the feet. This, in turn, lowers the deep body temperature T.

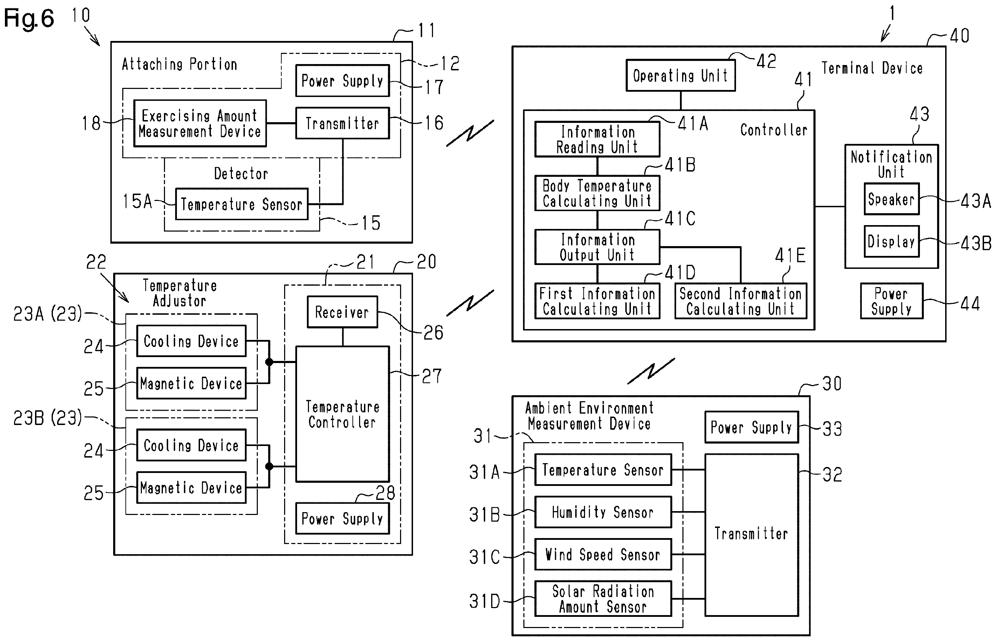

With reference to Fig. 6, the configuration of the exercise assistance system 1 will now be described in detail.

The deep body thermometer 10 further includes a transmitter 16 and a power supply 17. The transmitter 16 transmits first state information that is detected by the detector 15. The first state information includes information related to the deep body temperature T. In one example, the transmitter 16 transmits the first state information, which is detected by the temperature sensor 15A, to the terminal device 40. The power supply 17 supplies power to various types of electrical elements in the deep body thermometer 10. The deep body thermometer 10 further includes an exercising amount measurement device 18 that allows for measurement of an exercising amount. The exercising amount measurement device 18 is configured to measure, for example, a fatigue-related exercising amount of the person who is exercising. The fatigue-related exercising amount of the person who is exercising includes exercising load on the person who is exercising, moving distance of the person who is exercising, and the like. The exercising amount measurement device 18 includes at least one of an acceleration sensor and a distance sensor. The exercising amount measurement device 18 may be separate from the deep body thermometer 10.

The temperature adjustor 20 includes a receiver 26, a temperature controller 27, and a power supply 28. The receiver 26 receives various types of information from the terminal device 40. In one example, the receiver 26 receives various types of information related to control of the adjustment equipment 22. The various types of information related to control of the adjustment equipment 22 includes at least one of information related to the temperature of the cooling medium in the cooling device 24, information related to a magnetic stimulating pattern of the magnetic device 25, and information related to the driving time of the temperature adjustor 20. In the present embodiment, the receiver 26 receives information transmitted from a controller 41 of the terminal device 40 that executes an exercise assistance program. The temperature controller 27 controls the adjustment equipment 22 based on, for example, various types of information received from the receiver 26. In one example, the temperature controller 27 controls the cooling device 24 and the magnetic device 25 to adjust the body temperature in accordance with the information received by the receiver 26. The power supply 28 supplies power to various types of electrical elements in the temperature adjustor 20. An operating unit for operating the temperature adjustor 20 without the terminal device 40 may be arranged on the temperature adjustor 20.

The exercise assistance system 1 further includes an ambient environment measurement device 30. The ambient environment measurement device 30 is configured to measure information related to the ambient environment (hereafter referred to as the environment information). The ambient environment measurement device 30 is configured to be electrically connectable to the terminal device 40 through wired or wireless communication. In one example, the ambient environment measurement device 30 is configured to be connectable to the terminal device 40 through wireless communication. The main elements of the ambient environment measurement device 30 include a detector 31, a transmitter 32, and a power supply 33. The detector 31 includes at least one of a temperature sensor 31A that detects the ambient temperature, a humidity sensor 31B that detects the humidity, a wind speed sensor 31C that detects the wind speed, and a solar radiation amount sensor 31D that detects the solar radiation amount. The transmitter 32 transmits the environment information that is detected by the detector 31. In one example, the transmitter 32 transmits the environment information, which is detected by the various sensors of the detector 31, to the terminal device 40. The power supply 33 supplies power to various types of electrical elements in the ambient environment measurement device 30.

The main elements of the terminal device 40 include the controller 41, an operating unit 42, a notification unit 43, and a power supply 44. The controller 41 includes one or more central processing units (CPUs) or micro-processing units (MPU). The controller 41 may be configured as circuity including 1) one or more processors that run on computer programs (software) to execute various processes, 2) one or more dedicated hardware circuits such as application-specific integrated circuits (ASICs) that execute at least some of the processes, or 3) a combination of processors and hardware circuits. The processors include a CPU and a memory such as a RAM and a ROM, and the memory stores program codes or instructions configured to have the CPU execute processes. The memory, namely, a computer readable medium, includes any available medium that is accessible by a versatile or dedicated computer. The controller 41 executes various types of control based on the first state information and operation information related to operation of the operating unit 42. The controller 41 includes an information reading unit 41A, a body temperature calculating unit 41B, an information output unit 41C, a first information calculating unit 41D, and a second information calculating unit 41E. The information reading unit 41A reads the first state information related to the deep body temperature T detected by the detector 15. The information reading unit 41A outputs the first state information to the body temperature calculating unit 41B.

The body temperature calculating unit 41B calculates the deep body temperature T that corresponds to the first state information. In one example, the body temperature calculating unit 41B calculates the deep body temperature T based on detection results of the thermistor and thermopile that are included in the temperature sensor 15A.

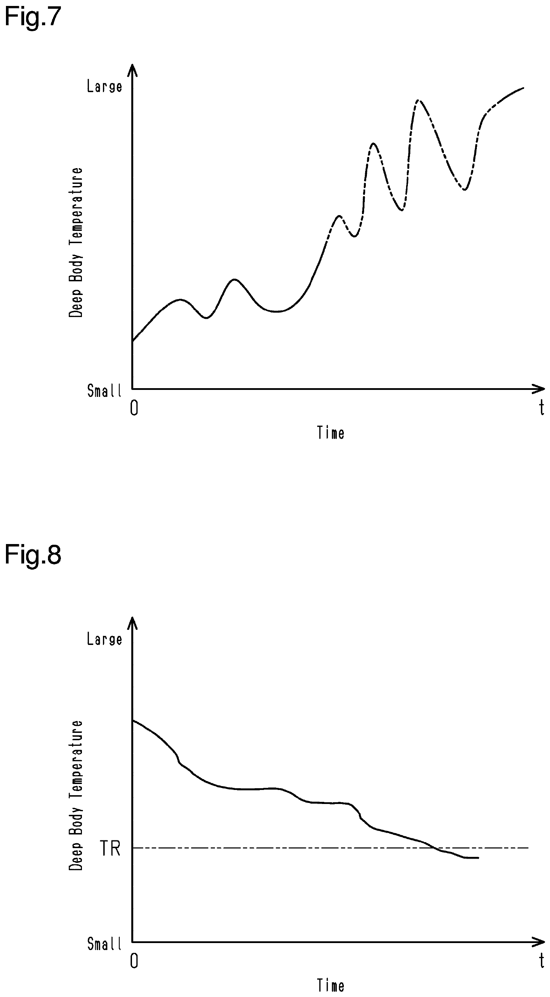

As shown in Fig. 7, the body temperature calculating unit 41B calculates a predicted deep body temperature T from the first state information. In one example, the body temperature calculating unit 41B calculates the predicted deep body temperature T from at least one of the first state information, information related to the exercising amount of the person who is exercising, and the environment information. The solid line in the graph of Fig. 7 shows one example of the deep body temperature T. The double-dashed line in the graph of Fig. 7 shows one example of the predicted deep body temperature T. The body temperature calculating unit 41B calculates an exercise-related performance from the predicted deep body temperature T. The exercise-related performance is correlated to the deep body temperature T. In one example, the exercise-related performance decreases as the predicted deep body temperature T rises. The body temperature calculating unit 41B takes this point into consideration to calculate the exercise-related performance.

As shown in Fig. 6, the body temperature calculating unit 41B outputs information related to the deep body temperature T to the information output unit 41C. The information output unit 41C outputs second state information related to the calculated deep body temperature T. In one example, the information output unit 41C outputs the second state information to the first information calculating unit 41D and the second information calculating unit 41E.

The first information calculating unit 41D calculates first instruction information from the second state information to control the temperature adjustor 20 that adjusts the body temperature. When, for example, the deep body temperature T is not included in a predetermined temperature range, the first information calculating unit 41D calculates the first instruction information from the second state information. The predetermined temperature range includes, for example, a range that is less than or equal to a reference temperature TR (refer to Fig. 8). The reference temperature TR is set based on, for example, the level of the exercise-related performance. In one example, when the deep body temperature T is included in the range that is less than or equal to the reference temperature TR, the exercise-related performance is relatively high. The first information calculating unit 41D may calculate the first instruction information from the second state information even when the deep body temperature T is included in the predetermined temperature range.

The first information calculating unit 41D calculates the first instruction information, which is used to operate the temperature adjustor 20, so that the deep body temperature T will be included in the predetermined temperature range. In one example, the first information calculating unit 41D calculates the first instruction information, which is used to operate the temperature adjustor 20, so that the deep body temperature T will be included in the predetermined temperature range within a predetermined period. The first information calculating unit 41D outputs the calculated first instruction information to the information output unit 41C. The information output unit 41C outputs the first instruction information to the temperature adjustor 20. The temperature controller 27 controls the cooling device 24 and the magnetic device 25 in accordance with the first instruction information. As shown in Fig. 8, the operation of the temperature adjustor 20 lowers the body temperature and the deep body temperature T. In one example, the operation of the temperature adjustor 20 ends when the deep body temperature T becomes less than or equal to the reference temperature TR.

As shown in Fig. 6, the second information calculating unit 41E calculates second instruction information from the second state information to control the notification unit 43 that issues notifications on information related to the deep body temperature T. The second information calculating unit 41E calculates the second instruction information from the second state information when, for example, the deep body temperature T is not included in the predetermined temperature range. The second information calculating unit 41E outputs the calculated second instruction information to the information output unit 41C. The information output unit 41C outputs the second instruction information to the notification unit 43. The notification unit 43 issues a notification of information related to the deep body temperature T corresponding to the second instruction information. In one example, the notification unit 43 issues a notification of at least one of the information related to the deep body temperature T measured by the deep body thermometer 10 and the information related to the predicted deep body temperature T calculated by the body temperature calculating unit 41B. The second information calculating unit 41E may calculate the second instruction information from the second state information even when the deep body temperature T is included in the predetermined temperature range.

The operating unit 42 is configured to allow input of information related to, for example, operation of the exercise assistance system 1. The notification unit 43 is configured to issue a notification related to, for example, information related to the exercise assistance system 1. The notification unit 43 includes at least one of a speaker 43A and a display 43B. The speaker 43A uses sound to issue a notification of information related to the exercise assistance system 1. The display 43B uses graphics to issue a notification of information related to the exercise assistance system 1. The display 43B may be integrated with the operating unit 42. In this case, the display 43B is a touch panel. The power supply 44 supplies power to various types of electrical elements of the terminal device 40.

The exercise assistance system 1 runs on an exercise assistance program to execute various types of control. The exercise assistance program has the controller 41 execute a first step S1 of reading the first state information related to the deep body temperature T detected by the detector 15, a second step S2 of calculating the deep body temperature T from the first state information, and a third step S3 of outputting the second state information related to the calculated deep body temperature T. The exercise assistance program allows the deep body temperature T to be measured.

In the second step S2, a predicted deep body temperature T is calculated from the first state information. The exercise assistance program presents the person who is exercising and his or her trainer or the like with information related to the predicted deep body temperature T. In the second step S2, the exercise-related performance is calculated from the predicted deep body temperature T. The exercise assistance program presents the person who is exercising and his or her trainer or the like with exercise-related information that is based on the deep body temperature T.

The exercise assistance program further has the controller 41 execute a fourth step of calculating the first instruction information from the second state information to control the temperature adjustor 20 that adjusts the body temperature. In the third step, the first instruction information further outputs the first instruction information. In the fourth step S4, the first instruction information is calculated to operate the temperature adjustor 20 so that the deep body temperature T will be included in the predetermined temperature range. The exercise assistance program improves the exercising performance. In the fourth step S4, the first instruction information is calculated to operate the temperature adjustor 20 so that the deep body temperature T will be included in the predetermined temperature range within a predetermined period. The exercise assistance program allows the deep body temperature T to be included in the predetermined temperature range within a limited length of time.

The exercise assistance program calculates the second instruction information from the second state information to control the notification unit 43 that issues a notification of information related to the deep body temperature T. In the third step, the second instruction information is further output. The exercise assistance program presents the person who is exercising and his or her trainer or the like with information related to the calculated deep body temperature T.

The exercise assistance system 1 runs on the exercise assistance program to execute, for example, at least one of a first control and a second control. The first control includes control for outputting the first instruction information. The second control includes control for outputting the second instruction information. In one example, at least one of the first control and the second control is executed based on operation of the operating unit 42. The exercise assistance system 1 may execute the first control and the second control in parallel under the exercise assistance program.

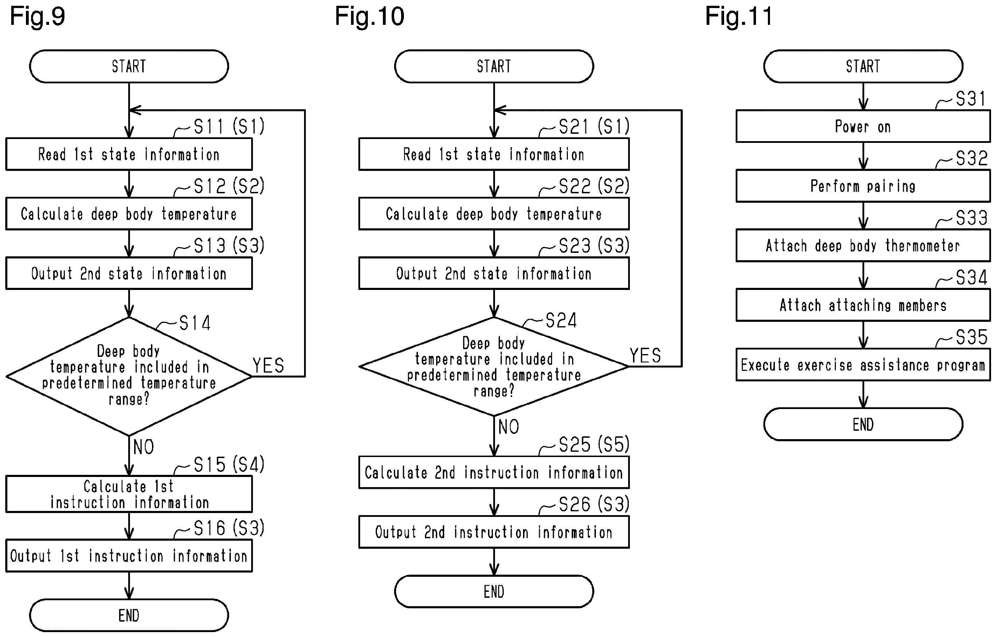

With reference to Fig. 9, one example of the first control executed by the exercise assistance system 1 will now be described.

In step S11, the controller 41 reads the first state information. Specifically, the information reading unit 41A reads the first state information related to the deep body temperature T detected by the temperature sensor 15A. Step S11 corresponds to the first step S1. In step S12, the controller 41 calculates the deep body temperature T. Specifically, the body temperature calculating unit 41B calculates the deep body temperature T from the first state information. In other words, the deep body temperature T is measured using the first state information. Step S12 corresponds to the second step S2. In step S13, the controller 41 outputs the second state information. Specifically, the information output unit 41C outputs the second state information related to the deep body temperature T calculated in step S12 to the first information calculating unit 41D. Step S13 corresponds to the third step S3.

In step S14, the controller 41 determines whether the deep body temperature T is included in the predetermined temperature range. When the controller 41 determines that the deep body temperature T is included in the predetermined temperature range in step S14, the controller 41 returns to step S11. When the controller 41 determines that the deep body temperature T is included in the predetermined temperature range in step S14, the controller 41 may end the first control. When the controller 41 determines that the deep body temperature T is not included in the predetermined temperature range in step S14, the controller 41 proceeds to step S15.

In step S15, the controller 41 calculates the first instruction information. Specifically, the first information calculating unit 41D calculates the first instruction information from the second state information. Step S15 corresponds to the fourth step S4. In step S16, the controller 41 outputs the first instruction information. Specifically, the information output unit 41C outputs the first instruction information to the temperature adjustor 20. Step S16 corresponds to the third step S3. The temperature controller 27 controls the cooling device 24 and the magnetic device 25 in accordance with the first instruction information.

This ends the process of steps S11 to step S16. The controller 41 may repeatedly execute the first control, which includes the process of steps S11 to step S16, during the period in which the exercise assistance system 1 is used. Step S14 may be omitted from the process of steps S11 to S16 illustrated in Fig. 9.

With reference to Fig. 10, one example of the second control executed by the exercise assistance system 1 will now be described.

The process of steps S21 to S22 included in the second control corresponds to the process of steps S11 to S12 included in the first control. In step S23, the controller 41 outputs the second state information. Specifically, the information output unit 41C outputs the second state information related to the deep body temperature T calculated in step S22 to the second information calculating unit 41E. Step S23 corresponds to the third step S3.

In step S24, the controller 41 determines whether the deep body temperature T is included in the predetermined temperature range. When determining that the deep body temperature T is included in the predetermined temperature range in step S24, the controller 41 returns to step S21. When determining that the deep body temperature T is not included in the predetermined temperature range in step S24, the controller 41 proceeds to step S25.

In step S25, the controller 41 calculates the second instruction information. Specifically, the second information calculating unit 41E calculates the second instruction information from the second state information. Step S25 corresponds to a fifth step S5. In step S26, the controller 41 outputs the second instruction information. Specifically, the information output unit 41C outputs the second instruction information to the notification unit 43. Step S26 corresponds to the third step S3. The notification unit 43 issues a notification of information related to the deep body temperature T corresponding to the second instruction information.

This ends the process of steps S21 to step S26. The controller 41 may repeatedly execute the second control, which includes the process of steps S21 to step S26, during the period in which the exercise assistance system 1 is used. Step S24 may be omitted from the process of steps S21 to S26 illustrated in Fig. 10.

With reference to Fig. 11, one example of how to use the exercise assistance system 1 will now be described.

The person who is exercising uses the exercise assistance system 1 during training or in a training interval in accordance with the steps described below. An example in which the exercise assistance system 1 is used in a training interval will now be described. In the example illustrated in Fig. 11, the first control is executed with the exercise assistance program.

In step S31, the person who is exercising turns on the power of the exercise assistance system 1. Activation of the exercise assistance system 1 calibrates the reference value of the temperature sensor 15A. In step S32, the person who is exercising pairs the terminal device 40 with each of the deep body thermometer 10, the temperature adjustor 20, and the ambient environment measurement device 30. In one example, the operating unit 42 is operated to pair the terminal device 40 with each of the deep body thermometer 10, the temperature adjustor 20, and the ambient environment measurement device 30. In step S33, the person who is exercising attaches the deep body thermometer 10 to his or her auricle. In step S34, the person who is exercising attaches the attaching members 23 to his or her body. In step S35, the exercise assistance program is executed. In one example, the controller 41 runs on the exercise assistance program and executes the first control. In the first control, the temperature adjustor 20 is controlled in accordance with the first instruction information. When the exercise assistance system 1 is used during training, the deep body thermometer 10 is attached to the auricle prior to training. Preferably, in this case, the notification unit 43 issues a notification of the time when the temperature adjustor 20 is controlled so that the attaching members 23 are attached to the body at that time.

The exercise assistance system 1 runs on the exercise assistance program to execute various types of control. This minimizes rises in the deep body temperature T. Thus, the exercise-related performance can be managed in a preferred manner. Further, when the second control is executed under the exercise assistance program, the person who is exercising or the trainer is provided with information related to the deep body temperature T. The trainer can instruct the person who is exercising on how to train, when to take a rest, and so on based on the information related to the deep body temperature T. This allows the exercise-related performance to be maintained.

### Modified Examples

The description related with the above embodiment exemplifies, without any intention to limit, applicable forms of an exercise assistance program, an exercise assistance system, and an exercise assistance system control method according to the present invention. The exercise assistance program, the exercise assistance system, and the exercise assistance system control method according to the present disclosure is applicable to, for example, modified examples of the above embodiment that are described below and combinations of at least two of the modified examples that do not contradict each other.

The controller 41 may be located anywhere. In a first example, the controller 41 is included in a server. In this case, the deep body thermometer 10, the temperature adjustor 20, and the ambient environment measurement device 30 each communicate with the terminal device 40 via the internet. In a second example, the controller 41 is arranged in the temperature adjustor 20. Specifically, the controller 41 is arranged in one of the main body 21 and the adjustment equipment 22. In this case, the main body 21 is configured to be electrically connectable to the controller 41 through wired or wireless communication. The adjustment equipment 22 is configured to be electrically connectable to the controller 41 through wired or wireless communication. When the controller 41 is arranged in the main body 21, the controller 41 may be integrated with or separate from the temperature controller 27.

The subject to which the attaching members 23 are attached may be changed freely. In a first example, the attaching members 23 includes bands that are attachable to at least either one of the wrists and the ankles. In a second example, the attaching members 23 includes a headband that is attachable to the head. In a third example, the attaching members 23 include a band that is attachable around the abdomen. In a fourth example, the attaching members 23 include a shirt that is wearable on the upper part of the body. In a fifth example, the attaching members 23 includes supporters that are attachable to the legs.

The configuration of the adjustment equipment 22 may be changed in any manner. In a first example, the adjustment equipment 22 does not include the cooling device 24. In a second example, the adjustment equipment 22 does not include the magnetic device 25. In a third example, the adjustment equipment 22 includes an electric simulating device in addition to or instead of at least one of the cooling device 24 and the magnetic device 25. The electric simulating device is configured to applied electric stimuli to the body.

The configuration of the exercise assistance system 1 may be changed in any manner. In a first example, the exercise assistance system 1 does not include the exercising amount measurement device 18. In a second example, the exercise assistance system 1 does not include the ambient environment measurement device 30. In this case, the exercise assistance system 1 may obtain the environment information via the internet. In a third example, the exercise assistance system 1 does not include one of the deep body thermometer 10 and the temperature adjustor 20. In a fourth example, the exercise assistance system 1 implements artificial intelligence (Al). In other words, the exercise assistance program includes artificial intelligence. Artificial intelligence includes, for example, deep learning using a neural network having a multilayer structure.

### Industrial Applicability

The exercise assistance program, the exercise assistance system, and the exercise assistance system control method according to the present invention can be applied to various types of household and commercial exercise assistance systems.

## Claims

1. An exercise assistance system comprising:
a deep body thermometer (10) configured to be attached to a user and including a temperature sensor (15A);
a temperature adjustment equipment (20) configured to be attached to at least one of a hand and a foot of the user; and
a controller (41) configured to communicate with the deep body thermometer (10) and the temperature adjustment equipment (20), wherein
the deep body thermometer (10) is configured to detect information related to deep body temperature of the user with the temperature sensor (15A),
the controller (41) is configured to execute a process for determining whether the deep body temperature of the user is less than or equal to a predetermined temperature based on the detected information related to deep body temperature, and
**characterized in that**
the temperature adjustment equipment (20) includes a magnetic device (25) configured to apply magnetic stimuli that dilate blood vessels,
the magnetic device (25) includes stimulating portions (25A) configured to apply the magnetic stimuli, and
when the controller (41) determines that the deep body temperature is not less than or equal to the predetermined temperature, the controller (41) is configured to execute a process for outputting control information to the temperature adjustment equipment (20) to apply the magnetic stimuli to a palm or foot bottom of the user.

2. A method for controlling an exercise assistance system (1), wherein the exercise assistance system (1) includes a deep body thermometer (10), which is attached to a user and includes a temperature sensor (15A), and a temperature adjustment equipment (20), which is attached to at least one of a hand and a foot of the user, wherein the temperature adjustment equipment (20) includes a magnetic device (25) configured to apply magnetic stimuli that dilate blood vessels, and the magnetic device (25) includes stimulating portions (25A) configured to apply the magnetic stimuli, the method comprising:
detecting information related to deep body temperature of the user with the temperature sensor (15A);
determining whether the deep body temperature of the user is less than or equal to a predetermined temperature based on the detected information related to deep body temperature, and
**characterized by**
when it is determined that the deep body temperature is not less than or equal to the predetermined temperature, outputting control information to apply the magnetic stimuli to a palm or foot bottom of the user.

3. An exercise assistance program comprising instructions to cause the controller of claim 1 to execute the steps of the method of claim 2.

## Patentansprüche

1. System für Unterstützung bei Übungen, das umfasst:
ein Körperkerntemperatur-Thermometer (10), das zum Anbringen an einem Benutzer ausgeführt ist und einen Temperatursensor (15A) enthält;
eine Einrichtung (20) für Temperatureinstellung, die zum Anbringen an einer Hand oder/und einem Fuß des Benutzers ausgeführt ist; sowie
eine Steuerungseinrichtung (41), die zum Kommunizieren mit dem Körperkerntemperatur-Thermometer (10) und der Einrichtung (20) für Temperatureinstellung ausgeführt ist, wobei
das Körperkerntemperatur-Thermometer (10) zum Erfassen von Informationen, die sich auf die Körperkerntemperatur des Benutzers beziehen, mit dem Temperatursensor (15A) ausgeführt ist,
die Steuerungseinrichtung (41) zum Ausführen eines Prozesses ausgeführt ist, mit dem auf Basis der erfassten Informationen, die sich auf die Körperkerntemperatur beziehen, festgestellt wird, ob die Körperkerntemperatur des Benutzers unter oder auf einer vorgegebenen Temperatur liegt, und
**dadurch gekennzeichnet, dass**
die Einrichtung (20) für Temperatureinstellung eine magnetische Vorrichtung (25) enthält, die zum Ausüben magnetischer Stimuli ausgeführt ist, die Blutgefäße aufweiten,
die magnetische Vorrichtung (25) stimulierende Abschnitte (25A) enthält, die zum Ausüben der magnetischen Stimuli ausgeführt sind, und
die Steuerungseinrichtung (41) so ausgeführt ist, dass sie, wenn die Steuerungseinrichtung (41) feststellt, dass die Körperkerntemperatur nicht unter oder auf der vorgegebenen Temperatur liegt, einen Prozess zum Ausgeben von Steuerungsinformationen an die Einrichtung (20) für Temperatureinstellung zum Ausüben der magnetischen Stimuli auf eine Handfläche oder eine Fußsohle des Benutzers ausführt.

2. Verfahren zum Steuem eines Systems (1) für Unterstützung bei Übungen, wobei das System (1) für Unterstützung bei Übungen ein Körperkerntemperatur-Thermometer (10), das an einem Benutzer angebracht ist und einen Temperatursensor (15A) enthält, sowie eine Einrichtung (20) für Temperatureinstellung enthält, die an einer Hand oder/und einem Fuß des Benutzers angebracht ist, wobei die Einrichtung (20) für Temperatureinstellung eine magnetische Vorrichtung (25) enthält, die zum Ausüben magnetischer Stimuli ausgeführt ist, die Blutgefäße aufweiten, die magnetische Vorrichtung (25) stimulierende Abschnitte (25A) enthält, die zum Ausüben der magnetischen Stimuli ausgeführt sind, und das Verfahren umfasst:
Erfassen von Informationen, die sich auf die Körperkerntemperatur des Benutzers beziehen, mit dem Temperatursensor (15A);
Feststellen, ob die Körperkerntemperatur des Benutzers unter oder auf einer vorgegebenen Temperatur liegt, auf Basis der erfassten Informationen, die sich auf die Körperkerntemperatur beziehen, und
**gekennzeichnet durch**
Ausgeben von Steuerungsinformationen zum Ausüben der magnetischen Stimuli auf eine Handfläche oder eine Fußsohle des Benutzers, wenn festgestellt wird, dass die Körperkerntemperatur nicht unter oder auf der vorgegebenen Temperatur liegt.

3. Programm für Unterstützung bei Übungen, das Befehle umfasst, die die Steuerungseinrichtung nach Anspruch 1 veranlassen, die Schritte des Verfahrens nach Anspruch 2 auszuführen.

## Revendications

1. Système d'assistance à l'exercice comprenant :
un thermomètre de prise de température corporelle centrale (10) configuré pour être lié à un utilisateur et incluant un capteur de température (15A) ;
un équipement de réglage de température (20) configuré pour être lié à au moins un membre parmi une main et un pied de l'utilisateur ; et
un contrôleur (41) configuré pour communiquer avec le thermomètre de prise de température corporelle centrale (10) et l'équipement de réglage de température (20),
dans lequel :
le thermomètre de prise de température corporelle centrale (10) est configuré pour détecter une information se rapportant à la température corporelle centrale de l'utilisateur à l'aide du capteur de température (15A), et
le contrôleur (41) est configuré pour exécuter un processus pour déterminer si la température corporelle centrale de l'utilisateur est inférieure ou égale à une température prédéterminée sur la base de l'information détectée se rapportant à la température corporelle centrale, et
**caractérisé en ce que** :
l'équipement de réglage de température (20) inclut un dispositif magnétique (25) configuré pour appliquer des stimuli magnétiques qui dilatent les vaisseaux sanguins,
le dispositif magnétique (25) inclut des parties de stimulation (25A) configurées pour appliquer les stimuli magnétiques, et
lorsque le contrôleur (41) détermine que la température corporelle centrale n'est ni inférieure, ni égale à la température prédéterminée, le contrôleur (41) est configuré pour exécuter un processus pour émettre en sortie une information de commande sur l'équipement de réglage de température (20) afin d'appliquer les stimuli magnétiques sur une paume ou sur une base de pied de l'utilisateur.

2. Procédé pour commander un système d'assistance à l'exercice (1), dans lequel le système d'assistance à l'exercice (1) inclut un thermomètre de prise de température corporelle centrale (10), lequel est lié à un utilisateur et inclut un capteur de température (15A), et un équipement de réglage de température (20), lequel est lié à au moins un membre parmi une main et un pied de l'utilisateur, dans lequel l'équipement de réglage de température (20) inclut un dispositif magnétique (25) configuré pour appliquer des stimuli magnétiques qui dilatent les vaisseaux sanguins, et le dispositif magnétique (25) inclut des parties de stimulation (25A) configurées pour appliquer les stimuli magnétiques, le procédé comprenant :
la détection d'une information se rapportant à la température corporelle centrale de l'utilisateur à l'aide du capteur de température (15A) ;
la détermination de si la température corporelle centrale de l'utilisateur est inférieure ou égale à une température prédéterminée sur la base de l'information détectée se rapportant à la température corporelle centrale, et
**caractérisé par** :
lorsqu'il est déterminé que la température corporelle centrale n'est ni inférieure, ni égale à la température prédéterminée, l'émission en sortie d'une information de commande afin d'appliquer les stimuli magnétiques sur une paume ou sur une base de pied de l'utilisateur.

3. Programme d'assistance à l'exercice comprenant des instructions pour forcer le contrôleur selon la revendication 1 à exécuter les étapes du procédé selon la revendication 2.
